Europäisches Patentamt

European Patent Office

Office européen des brevets

(11) Veröffentlichungsnummer : **0 356 665 B1**

(12) # EUROPÄISCHE PATENTSCHRIFT

(45) Veröffentlichungstag der Patentschrift :
**03.06.92 Patentblatt 92/23**

(51) Int. Cl.⁵ : **A61K 7/09**

(21) Anmeldenummer : **89112631.0**

(22) Anmeldetag : **11.07.89**

(54) **Kationisches Fixiermittel und Verfahren zur dauerhaften Haarverformung.**

(30) Priorität : **03.08.88 DE 3826369**

(43) Veröffentlichungstag der Anmeldung :
**07.03.90 Patentblatt 90/10**

(45) Bekanntmachung des Hinweises auf die
Patenterteilung :
**03.06.92 Patentblatt 92/23**

(84) Benannte Vertragsstaaten :
**AT BE CH DE ES FR GB GR IT LI NL SE**

(56) Entgegenhaltungen :
**DE-A- 3 535 351**
**US-A- 4 220 167**
**CHEMICAL ABSTRACTS, Band 89, Nr. 6, Seite**
**310, Zusammenfassung Nr. 48802c, Colum-**
**bus, Ohio, US**

(73) Patentinhaber : **Wella Aktiengesellschaft**
**Berliner Allee 65**
**W-6100 Darmstadt (DE)**

(72) Erfinder : **Götz, Harry**
**Waldstrasse 21**
**W-6146 Alsbach-Hähnlein 2 (DE)**
Erfinder : **Hartmann, Peter**
**Hoffmannstrasse 6**
**W-6100 Darmstadt (DE)**
Erfinder : **Köhler, Joachim, Dr.**
**Waldstrasse 51**
**W-6101 Brensbach/Odw. (DE)**
Erfinder : **Lang, Günther, Dr.**
**Auf der Roten Erde 10 B**
**W-6107 Reinheim 5 (DE)**

## Beschreibung

Die vorliegende Erfindung betrifft ein Fixiermittel für die Durchführung der oxidativen Nachbehandlung bei der dauerhaften Haarverformung, das eine Mischung aus einem Oxidationsmittel, einem kationischen Polymer und/oder einem kationischen Tensid sowie einem Alkali- oder Ammoniumsalz der 2-Pyrrolidon-5-carbonsäure enthält, sowie ein Verfahren zur dauerhaften Haarverformung unter Verwendung dieses Fixiermittels.

Bei der dauerhaften Haarverformung werden die Haare in der Regel zunächst mit einem Verformungsmittel auf der Basis einer keratinreduzierenden Mercaptoverbindung, welche eine Öffnung der Disulfidbrückenbindungen des Haarkeratins bewirkt, behandelt und sodann in die gewünschte Form gebracht. Anschließend wird das Haar mit Wasser gespült und sodann mit einem Fixiermittel oxidativ nachbehandelt. Hierbei werden die zuvor gespaltenen Disulfidbrückenbindungen in der neuen Form wiederverknüpft.

Durch die bei dieser Verfahrensweise auf das Haar einwirkenden chemischen und mechanischen Vorgänge wird die Haarqualität, insbesondere bezüglich Griff und Kämmbarkeit des Haares, verschlechtert und das Haar wird spröde und rauh.

Es wurde bereits versucht, durch Zusatz bestimmter pflegender Substanzen zu dem Verformungsmittel oder dem Fixiermittel, dem Haar einen glatten, angenehmen Griff zu verleihen sowie die Kämmbarkeit der Haare zu verbessern. Als besonders vorteilhaft hat sich hierbei die Verwendung von kationischen Verbindungen, beispielsweise kationischen Polymeren und kationischen Tensiden, in Fixiermitteln erwiesen. Andererseits werden durch die Verwendung von kationischen Verbindungen in Fixiermitteln jedoch das Umformungsergebnis und die Ausspülbarkeit des Fixiermittels verschlechtert.

Es bestand daher die Aufgabe, ein Fixiermittel für die Durchführung der oxidativen Nachbehandlung bei der dauerhaften Haarverformung zur Verfügung zu stellen, das eine gute Konditionierung der Haare ermöglicht, mit Wasser leicht ausspülbar ist und ein gutes Verformungsergebnis gewährleistet.

Überraschenderweise wurde nunmehr gefunden, daß die gestellte Aufgabe durch ein Fixiermittel, welches einen oxidierenden Wirkstoff, ein kationisches Polymer und/oder ein kationisches Tensid sowie ein Salz der 2-Pyrrolidon-5-carbonsäure enthält, in hervorragender Weise gelöst wird.

Gegenstand der vorliegenden Erfindung ist daher ein Fixiermittel für die Durchführung der oxidativen Nachbehandlung bei der dauerhaften Haarverformung auf der Basis eines oxidierenden Wirkstoffes sowie eines kationischen Polymers und/oder eines kationischen Tensides, welches dadurch gekennzeichnet ist, daß es ein Alkali- oder Ammoniumsalz der 2-Pyrrolidon-5-carbonsäure enthält.

Das erfindugnsgemäße Fixiermittel enthält vorzugsweise 0,05 bis 5 Gewichtsprozent eines Alkali- oder Ammoniunsalzes der 2-Pyrrolidon-5-carbonsäure.

Als Alkali- oder Ammoniumsalz der 2-Pyrrolidon-5-carbonsäure kommen insbesondere Natrium-2-pyrrolidon-5-carboxylat, Kalium-2-pyrrolidon-5-carboxylat und Ammonium-2-pyrrolidon-5-carboxylat in Betracht, wobei das Natrium-2-pyrrolidon-5-carboxylat besonders bevorzugt ist.

Das Fixiermittel enthält weiterhin ein kationisches Polymer und/oder ein kationisches Tensid, vorzugsweise in einer Gesamtmenge von 0,1 bis 5 Gewichtsprozent.

Für die Verwendung in dem erfindungsgemäßen Fixiermittel sind beispielsweise die folgenden kationischen Polymere oder Mischungen dieser kationischen Polymere geeignet: Kationische Cellulosederivate, wie zum Beispiel kationische Celluloseether (beispielsweise CTFA-Polyquaternium-10), mit Methylbromid quaternisierte Polydimethylaminoethylmethacrylate (CTFA-Polyquaterium-9), Polydimethylaminoethylmethacrylat (zu 75 % mit Dimethylsulfat quaternisiert), Polydiallyldimethylammoniumchlorid (CTFA-Polyquaterium-6), Diallyldimethylammoniumchlorid/Hydroxyethylcellulose-Copolymere (CTFA-Polyquaternium-4), Beta-methyacryloxyethyl-trimethylammoniummethosulfat-Homopolymere (CTFA-Polyquaternium-14), beta-Methacryloxyethyl-trimethylammoniummethosulfat/Acrylamid-Copolymere (CTFA-Polyquaternium-5), beta-Methacryloxyethyl-trimethylammoniummethosulfat/Vinylpyrrolidon-Copolymere (CTFA-Polyquaternium-11), Diallyldimethylammoniumchlorid/Acrylamid-Copolymere (CTFA-Polyquaternium-7) und kationische Chitosanderivate. Die vorstehend genannten Polyquaternium-Verbindungen werden im CTFA Cosmetic Ingredient Dictionary, 3rd Edition (1982), auf Seite 245 beschrieben.

Unter diesen kationischen Polymeren sind die folgenden Verbindungen besonders bevorzugt: Polydialkyldimethylammoniumchlorid, Polydimethylaminoethylmethacrylat (zu 75 % mit Dimethylsulfat quaternisiert), beta-Methacryloxyethyl-trimethylammoniummethosulfat/Vinylpyrrolidon-Copolymere und beta-Methacryloxyethyl-trimethylammoniummethosulfat-Homopolymere.

Als geeignete Tenside sind insbesondere kationische oberflächenaktive Agenzien, wie beispielsweise Dilauryldimethylammoniumchlorid, Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, Dialkyldimethylammoniumchloride oder -bromide, beispielsweise Dicetyldimethylammoniumchlorid, Alkylpyridiniumsalze, bei-

2

spielsweise Lauryl- oder Cetylpyridiniumchlorid, oder Alkylamidoethyltrimethylammoniumethersulfate zu nennen, wobei Cetyltrimethylammoniumchlorid und Dicetyldimethylammoniumchlorid besonders bevorzugt sind.

Als oxidierender Wirkstoff kann jede beliebige, bisher in Fixiermitteln verwendete oxidierende Verbindung eingesetzt werden. Beispiele für oxidierende Wirkstoffe sind Alkalibromate, wie zum Beispiel Kalium- und Natriumbromat, oder Wasserstoffperoxid. Die in dem Fixiermittel enthaltene Menge des oxidierenden Wirkstoffes ist in Abhängigkeit von der Anwendungsdauer und Anwendungstemperatur unterschiedlich. Normalerweise wird der oxidierende Wirkstoff in einer Menge von etwa 0,1 bis 15 Gewichtsprozent verwendet.

Das Fixiermittel kann in Form einer wäßrigen Lösung oder Emulsion sowie in verdickter Form auf wäßriger Basis, insbesondere als Creme, Gel oder Paste vorliegen. Ebenfalls ist es möglich, das Fixiermittel gemeinsam mit einem unter Druck verflüssigten Treibgas in einem Druckbehälter abzufüllen und daraus als Schaum zu entnehmen.

Der pH-Wert des gebrauchsfertigen Fixiermittels beträgt in Abhängigkeit vom verwendetem oxidierenden Wirkstoff etwa 1,5 bis 8,5. So beträgt der pH-Wert bei Verwendung von Wasserstoffperoxid als oxidierenden Wirkstoff vorzugsweise 1,5 bis 6, während bei Verwendung von Alkalibromaten ein pH-Wert von 6 bis 8,5 bevorzugt ist.

Selbstverständlich kann das Fixiermittel alle für derartige Mittel üblichen Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, Netzmittel oder Emulgatoren aus den Klassen der anionischen, amphoteren oder nichtionogenen oberflächenaktiven Substanzen, beispielsweise Fettalkoholsulfate, Fettalkoholethersulfate, Alkylsulfonate, Alkylbenzolsulfate, Alkylbetaine, oxethylierte Fettalkohole, oxethylierte Alkylphenole, Fettsäurealkanolamide oder oxethylierte Fettsäureester, ferner Farbstoffe, Trübungsmittel, wie zum Beispiel Polyethylenglykolester, oder Alkohole, wie beispielsweise Ethanol, Propanol und Isopropanol, Lösungsvermittler, Puffersubstanzen, Parfümöle sowie haarkonditionierende oder haarpflegende Bestandteile, wie zum Beispiel Lanolinderivate, Cholesterin oder Betain, enthalten.

Weiterhin können dem Fixiermittel die üblichen Quell- und Penetrationsstoffe, wie zum Beispiel Harnstoff, 2-Pyrrolidon, 1-Methyl-2-pyrrolidon und Dipropylenglykolmonomethylether sowie Peroxidstabilisatoren, beispielsweise aromatische Sulfonsäuren, ortho-Phosphorsäure, Poly- oder Pyrophosphorsäuren, saure Salze starker Säuren, organische Säuren, Phenacetin, Phenol, Thymol oder alpha-Bisabolol, zugesetzt werden.

Bei Verwendung des vorstehend beschriebenen Fixiermittels für die oxidative Nachbehandlung bei der dauerhaften Haarverformung werden Griff und Kämmbarkeit des Haares verbessert und gleichzeitig das Gesamtumformungsergebnis günstig beeinflußt. Außerdem läßt sich das Fixiermittel im Vergleich zu üblichen kationischen Fixiermitteln mit Wasser wesentlich leichter aus dem Haar ausspülen.

Ein weiterer Gegenstand der vorliegenden Erfindung ist daher ein Verfahren zur dauerhaften Haarverformung, bei dem man das Haar bevor und/oder nachdem man es in die gewünschte Form bringt mit einem keratinreduzierenden Verformungsmittel behandelt, mit Wasser spült, sodann mit einem Fixiermittel oxidativ nachbehandelt, mit Wasser spült, anschließend zur Frisur legt und sodann trocknet, welches dadurch gekennzeichnet ist, daß für die oxidative Nachbehandlung das vorstehend beschriebene erfindungsgemäße Fixiermittel verwendet wird.

Bei dem erfindungsgemäßen Verfahren wird das Haar gewaschen, mit einem Handtuch frottiert, gegebenenfalls mit einem Teil des keratinreduzierenden Verformungsmittels vorgefeuchtet, in einzelne Strähnen aufgeteilt und auf Wickler gewickelt. Der Durchmesser der Wickler beträgt hierbei, je nachdem ob eine Dauerwellung oder eine Entkräuselung der Haare gewünscht wird, entweder 5 bis 13 Millimeter oder etwa 15 bis 35 Millimeter. Auf das gewickelte Haar wird anschließend eine für die dauerhafte Haarverformung ausreichende Menge des Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglycerin, Cysteamin, Cystein sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäure, wie beispielsweise Monothioglykolsäureglykolester- oder -glycerinester, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten.

Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können.

Das bei dem erfindungsgemäßen Verfahren verwendete Verformungsmittel kann sowohl in Form einer wäßrigen Lösung oder Emulsion als auch in verdickter Form auf wäßriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 20 Minuten mit Wärmeinwirkung; 20 bis 45 Minuten ohne Wärmeinwirkung) beträgt, wird das Haar mit Wasser gespült und sodann mit etwa 80 bis 120 Gramm des vorstehend beschriebenen erfindungsgemäßen Fixiermittels oxidativ nachbehandelt.

Nach einer Einwirkungszeit von etwa 1 bis 20 Minuten werden die Wickler entfernt und das abgewickelte Haar, falls erforderlich, nochmals mit dem Fixiermittel oxidativ nachbehandelt. Sodann wird das Haar mit Wasser gespült, zur Frisur gelegt und getrocknet.

Das so behandelte Haar besitzt eine gleichmäßige und haltbare Umformung und ist hervorragend konditioniert.

Die nachfolgenden beispiele sollen den Gegenstand der vorliegenden Erfindung näher erläutern, ohne den Gegenstand auf diese Beispiele zu beschränken.

**Beispiele**

**Beispiel 1: Fixiermittel auf Wasserstoffperoxidbasis**

```
2,50 g   Wasserstoffperoxid
0,30 g   Polydiallyldimethylammoniumchlorid
0,20 g   Cetyltrimethylammoniumchlorid
0,20 g   Natrium-2-pyrrolidon-5-carboxylat
2,00 g   Kokosfettsäuredimethylammonium-
         betain
0,30 g   ortho-Phosphorsäure
0,05 g   alpha-Bisabolol
94,45 g  Wasser (vollentsalzt)
100,00 g
```

Der pH-Wert dieses Fixiermittels beträgt 2,8.

Im Anschluß an die Behandlung der Haare mit einem Verformungsmittel auf Thioglykolatbasis wird das Haar mit Wasser gespült und sodann das vorstehend beschriebene Fixiermittel mit einem Schwamm aufgetragen. Nach einer Einwirkungszeit von 10 Minuten werden die Wickler entfernt und das Haar mit lauwarmem Wasser ausgespült. Anschließend wird das Haar zur Frisur gelegt und sodann getrocknet.

Das so behandelte Haar besitzt eine gleichmäßige Umformung und einen weichen Griff.

**Beispiel 2: Zweikomponenten-Fixiermittel**

| Komponente 1: | 12,0 g | Wasserstoffperoxid |
|---|---|---|
| | 1,0 g | ortho-Phosphorsäure |
| | 0,3 g | Rizinusöl, oxethyliert mit 40 Mol Ethylenoxid |
| | 0,3 g | Octylphenol, oxethyliert mit 20 Mol Ethylenoxid |
| | 0,1 g | alpha-Bisabolol |
| | <u>86,3 g</u> | Wasser (vollentsalzt) |
| | 100,0 g | |

| Komponente 2: | 20,0 g | beta-Methacryloxyethyl-trime- thylammoniummethosulfat-Homo- polymer |
|---|---|---|
| | 20,0 g | Kalium-2-pyrrolidon-5-carboxy- lat |
| | 40,0 g | |

Die Komponenten 1 und 2 werden kurz vor dem Gebrauch miteinander vermischt und das so erhaltene Konzentrat sodann mit 4 l Wasser verdünnt. Der pH-Wert dieses Fixiermittels beträgt 6,0.

Im Anschluß an eine Behandlung der Haare mit einem Verformungsmittel auf Thioglykolsäureester-Basis wird das Haar mit Wasser gespült und sodann mit dem vorstehend beschriebenen Fixiermittel unter Verwendung einer Umpumpvorrichtung 5 Minuten lang gespült. Nach Entfernung der Wickler wird das Haar mit Wasser gespült, zur Frisur gelegt und getrocknet.

**Beispiel 3: Fixiermittel auf Bromatbasis**

```
12,0 g   Natriumbromat
 0,5 g   beta-Methacryloxyethyl-trimethyl-
         ammoniummethosulfat/Vinylpyrroli-
         don-Copolymer
 0,4 g   Ammonium-2-pyrrolidon-5-carboxylat
 2,0 g   Isooctylphenol, oxethyliert mit
         10 Mol Ethylenoxid
 1,0 g   Dinatriumhydrogenphosphat x 12 H₂O
 0,7 g   Natriumdihydrogenphosphat x  2 H₂O
83,4 g   Wasser (vollentsalzt)
100,0 g
```

Das Fixiermittel besitzt einen pH-Wert von 6,3.

Im Anschluß an eine Haarwäsche wird das handtuchtrockene Haar auf Wickler mit einem Durchmesser von 10 Millimetern gewickelt und sodann mit 100 g einer 10-prozentigen wäßrigen Ammoniumthioglykolatlösung, deren pH-Wert mit Ammoniumcarbonat/Ammoniumhydrogencarbonat auf 9,0 eingestellt ist, gleichmäßig durchfeuchtet. Nach einer Einwirkungszeit von 20 Minuten bei Raumtemperatur wird das Haar mit Wasser gespült und sodann mit dem vorstehend beschriebenen Fixiermittel oxidativ nachbehandelt. Nach einer Einwirkungszeit von 10 Minuten werden die Wickler entfernt und die Haare, falls erforderlich, nochmals mit dem Fixiermittel behandelt und sodann mit Wasser gespült. Anschließend wird das Haar zur Frisur gelegt und getrocknet.

Das so behandelte Haar besitzt eine gleichmäßige Umformung und ist hinsichtlich Griff und Kämmbarkeit der Haare ausgezeichnet konditioniert.

**Patentansprüche**

1. Fixiermittel für die Durchführung der oxidativen Nachbehandlung bei der dauerhaften Haarverformung auf der Basis eines oxidierenden Wirkstoffes sowie eines kationischen Polymers und/oder eines kationischen Tensides, dadurch gekennzeichnet, daß es ein Alkali- oder Ammoniumsalz der 2-Pyrrolidon-5-carbonsäure enthält.

2. Fixiermittel nach Anspruch 1, dadurch gekennzeichnet, daß es 0,05 bis 5 Gewichtsprozent eines Alkali- oder Ammoniumsalzes der 2-Pyrrolidon-5-carbonsäure enthält.

3. Fixiermittel nach Anspruch 1 oder 2, dadurch gekennzeichnet, daß das Alkali- oder Ammoniumsalz der 2-Pyrrolidon-5-carbonsäure ausgewählt ist aus Natrium-2-Pyrrolidon-5-carboxylat, Kalium-2-pyrrolidon-5-carboxylat und Ammonium-2-pyrrolidon-5-carboxylat.

4. Fixiermittel nach einem der Ansprüche 1 bis 3, dadurch gekennzeichnet, daß es das kationische Polymer und/oder das kationische Tensid in einer Gesamtmenge von 0,1 bis 5 Gewichtsprozent enthält.

5. Fixiermittel nach einem der Ansprüche 1 bis 4, dadurch gekennzeichnet, daß das kationische Tensid ausgewählt ist aus Cetyltrimethylammoniumchlorid und Dicetyldimethylammoniumchlorid.

6. Fixiermittel nach einem der Ansprüche 1 bis 4, dadurch gekennzeichnet, daß das kationische Polymer ausgewählt ist aus Polydiallyldimethylammoniumchlorid, Polydimethylaminoethylmethacrylat (zu 75 % mit Dimethylsulfat quaternisiert), beta-Methacryloxyethyl-trimethylammoniummethosulfat/Vinylpyrrolidon-Copolymeren und beta-Methacryloxyethyl-trimethylammoniummethosulfat-Homopolymeren.

7. Fixiermittel nach einem der Ansprüche 1 bis 6, dadurch gekennzeichnet, daß es als oxidierenden Wirkstoff Wasserstoffperoxid enthält und einen pH-Wert von 1,5 bis 6 besitzt.

8. Fixiermittel nach einem der Ansprüche 1 bis 6, dadurch gekennzeichnet, daß es als oxidierenden Wirkstoff ein Alkali- oder Ammoniumbromat enthält und einen pH-Wert von 6 bis 8,5 besitzt.

EP 0 356 665 B1

9. Verfahren zur dauerhaften Haarverformung, bei dem man das Haar bevor und/oder nachdem man es in die gewünschte Form bringt mit einem keratinreduzierenden Verformungsmittel behandelt, mit Wasser spült, sodann mit einem Fixiermittel oxidativ nachbehandelt, mit Wasser spült, anschließend zur Frisur legt und sodann trocknet, dadurch gekennzeichnet, daß für die oxidative Nachbehandlung ein Fixiermittel nach einem der Ansprüche 1 bis 8 verwendet wird.

Claims

1. Fixing agent based on an oxidising agent as well as a cationic polymer and/or a cationic surfactant for the oxidative after-treatment step in the case of permanent hair shaping, characterised in that it contains an alkali or ammonium salt of 2-pyrrolidone-5-carboxylic acid.

2. Fixing agent according to Claim 1, characterised in that it contains 0.05 to 5 weight percent of an alkali or ammonium salt of 2-pyrrolidone-5-carboxylic acid.

3. Fixing agent according to Claim 1 or 2, characterised in that the alkali or ammonium salt of 2-pyrrolidone-5-carboxylic acid is selected from sodium-2-pyrrolidone-5-carboxylate, potassium-2-pyrrolidone-5-carboxylate and ammonium-2-pyrrolidone-5-carboxylate.

4. Fixing agent according to any one of Claims 1 to 3, characterised in that it contains the cationic polymer and/or the cationic surfactant in a total amount of 0.1 to 5 weight percent.

5. Fixing agent according to any one of Claims 1 to 4, characterised in that the cationic surfactant is selected from cetyltrimethylammoniumchloride and dicetyldimethylammoniumchloride.

6. Fixing agent according to any one of Claims 1 to 4, characterised in that the cationic polymer is selected from polydiallyldimethylammoniumchloride, polydimethylaminoethylmethacrylate (quarternised to 75% with dimethylsulphate), betamethacryloxyethyltrimethylammoniummethosulphate/vinypyrrolidone copolymers and betamethacryloxyethyltrimethylammoniummethosulphate homopolymers.

7. Fixing agent according to any one of Claims 1 to 6, characterised in that it contains hydrogen peroxide as the oxidising agent and has a pH value of 1.5 to 6.

8. Fixing agent according to any one of Claims 1 to 6, characterised in that it contains an alkali or ammonium bromate as the oxidising agent and has a pH value of 6 to 8.5.

9. Method for the permanent shaping of hair, in which before and/or after the hair has been arranged in the desired shape , it is treated with a keratin-reducing shaping agent, rinsed with water, subsequently treated oxidatively with a fixing agent, rinsed with water, subsequently set in a hair style and then dried, characterised in that a fixing agent according to any one of Claims 1 to 8 is used for the oxidative after-treatment step.

Revendications

1. Composition de fixation pour la mise en oeuvre d'un post-traitement oxydant lors d'une mise en plis permanente au moyen d'une matière active oxydante ainsi que d'un polymère cationique et/ou d'un agent tensioactif cationique, caractérisée en ce qu'elle contient un sel alcalin ou d'ammonium de l'acide 2-pyrrolidone-5-carboxylique.

2. Composition de fixation selon la revendication 1, caractérisée en ce qu'elle contient de 0,05 à 5 % en masse d'un sel alcalin ou d'ammonium de l'acide 2-pyrrolidone-5-carboxylique.

3. Composition de fixation selon la revendication 1 ou 2, caractérisée en ce que le sel alcalin ou d'ammonium de l'acide 2-pyrolidone-5-carboxylique est choisi parmi le carboxylate de sodium-2-pyrrolidone-5, le carboxylate de potassium-2-pyrrolidone-5, et le carboxylate d'ammonium-2-pyrrolidone-5.

4. Composition de fixation selon l'une des revendications 1 à 3, caractérisée en ce qu'elle contient le polymère cationique et/ou l'agent tensioactif cationique à raison de 0,1 à 5% en poids.

5. Composition de fixation selon l'une des revendications 1 à 4, caractérisé en ce que l'agent tensioactif cationique est choisi parmi le chlorure de cétyltriméthylammonium et le chlorure de dicétyldiméthylammonium.

6. Composition de fixation selon l'une des revendications 1 à 4, caractérisé en ce que le polymère cationique est choisi parmi le chlorure de polydiallyldiméthylammonium, le méthacrylate de polydiméthylaminoéthyle (quaternisé à 75% par du sulfate de diméthyle) des copolymères de méthosulfate de β-méthacryloxyéthyltriméthylammonium/vinylpyrrolidone, et des homopolymères de méthosulfate de β-méthacryloxytriméthyl-ammonium.

7. Composition de fixation selon l'une des revendications 1 à 6, caractérisé en ce qu'elle contient, comme substance active oxydante, du peroxyde d'hydrogène et présente une valeur de pH allant de 1,5 à 6.

8. Composition de fixation selon l'une des revendications 1 à 6, caractérisé en ce qu'elle contient comme

7

matière oxydante un bromate alcalin ou d'ammonium, et présente une valeur de pH allant de 6 à 8,5.

9. Procédé pour permanenter les cheveux, selon lequel, avant et/ou après avoir donné aux cheveux la forme souhaitée, on les traite avec un produit de déformation réduisant la kératine, on les rince à l'eau, puis on effectue un post-traitement oxydant avec un fixateur, on rince à l'eau et on fait la mise en plis et on sèche, caractérisé en ce qu'on utilise pour le post-traitement oxydant un fixateur selon l'une des revendications 1 à 8.